(19) Europäisches Patentamt / European Patent Office / Office européen des brevets

(11) **EP 3 786 600 B1**

(12) **EUROPEAN PATENT SPECIFICATION**

(45) Date of publication and mention
of the grant of the patent:
**15.03.2023 Bulletin 2023/11**

(21) Application number: **19791476.5**

(22) Date of filing: **25.03.2019**

(51) International Patent Classification (IPC):
$G01L\ 1/00^{(2006.01)}$    $G01L\ 1/25^{(2006.01)}$
$G01N\ 23/2055^{(2018.01)}$    $G01N\ 33/204^{(2019.01)}$
$G01L\ 5/00^{(2006.01)}$    $G06F\ 30/23^{(2020.01)}$

(52) Cooperative Patent Classification (CPC):
**G01N 33/204; G01L 1/25; G01L 5/0047;
G01N 23/2055; G06F 30/23;** G01N 2223/607

(86) International application number:
**PCT/JP2019/012534**

(87) International publication number:
**WO 2019/208061 (31.10.2019 Gazette 2019/44)**

(54) **METHOD FOR COMPUTING RESIDUAL STRESS**

VERFAHREN ZUR BERECHNUNG VON RESTSPANNUNG

PROCÉDÉ DE CALCUL DE CONTRAINTE RÉSIDUELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.04.2018 JP 2018083986**

(43) Date of publication of application:
**03.03.2021 Bulletin 2021/09**

(73) Proprietor: **Kabushiki Kaisha Kobe Seiko Sho
(Kobe Steel, Ltd.)
Kobe-shi, Hyogo 651-8585 (JP)**

(72) Inventors:
• **MATSUDA, Mariko**
  **Takasago-shi, Hyogo 676-8670 (JP)**
• **OKITA, Keisuke**
  **Kobe-shi, Hyogo 651-2271 (JP)**
• **NAKAGAWA, Tomokazu**
  **Kobe-shi, Hyogo 651-2271 (JP)**

(74) Representative: **Müller-Boré & Partner
Patentanwälte PartG mbB
Friedenheimer Brücke 21
80639 München (DE)**

(56) References cited:
**WO-A1-2016/140091    WO-A1-2016/140093
WO-A1-2016/140094    CN-A- 103 853 899
JP-A- 2005 181 172    JP-A- 2008 058 179
JP-A- 2015 145 834    US-A1- 2016 273 979**

**Description**

[TECHNICAL FIELD]

The present invention relates to a method for calculating a residual stress.

[BACKGROUND ART]

In a metallic structure, inherent strain(s) such as a thermal strain, a plastic strain, and/or the like are/is generated due to conditions of producing the structure. Inherent strain having been generated induces an elastic strain. Consequently, a residual stress originating from the inherent strain is generated in the structure.

The inherent strain generated in the structure does not change even when the structure is divided so as to release the elastic strain. In this context, there is known an inherent strain method in which a released elastic strain or a residual stress originating from an inherent strain is measured by appropriately dividing a structure, the inherent strain existing in the structure is appropriately ascertained by inverse analysis using the measured value, and a residual stress in the structure is calculated from the inherent strain which has been ascertained. For example, Patent Document 1 discloses a residual stress measuring method that enables analysis of a residual stress in a plate joint or an axisymmetric joint.

The residual stress measuring method disclosed in Patent Document 1 is a residual stress analysis method using a general inherent strain method. In this method, a residual stress is calculated in the following procedure. First, elastic strains in three directions are each measured by cutting a structure to be a target of the residual stress analysis. A coefficient matrix representing a relation between elastic strains and unit values of inherent strains is determined by finite element analysis. Most probable values of inherent strains in the three directions are determined using the elastic strains in the three directions which have been measured and the coefficient matrix which has been determined. Then, a most probable value of the residual stress is calculated using the coefficient matrix and the most probable values of the inherent strains.

In the residual stress measuring method of Patent Document 1, the coefficient matrix representing the relation between the inherent strain and the elastic strain is determined; however, calculation thereof requires great time and effort. Therefore, to reduce effort for the calculation, production of a dedicated program for automatically calculating the coefficient matrix by the finite element analysis can be considered. However, the production of the dedicated program requires expertise relating to a finite element method.

Furthermore, in the inherent strain method, to measure the residual stress or the elastic strain which is generated in the structure, an inside of the structure must be exposed as a cutting face by cutting the structure. However, a measurement error inevitably occurs in the measurement of the residual stress or the elastic strain on the cutting face and a surface of the structure. In addition, the inherent strain method requires an extremely large number of measurement points of the residual stress or the elastic strain: generally, several tens to several hundreds of measurement points are required. Accordingly, it is difficult to appropriately take the measurement error into consideration.

[PRIOR ART DOCUMENTS]

[PATENT DOCUMENTS]

Patent Document 1: Japanese Unexamined Patent Application, Publication No. 2005-181172

US 2016/273979 A1 relates to a method capable of accurately measuring, in an object including a columnar shaft section and a tabular section projecting outward radially beyond an outer circumferential surface of the shaft section, a distribution of residual stress near a fillet surface interconnecting the shaft section and the tabular section.

[SUMMARY OF THE INVENTION]

[PROBLEMS TO BE SOLVED BY THE INVENTION]

The present invention was made in view of the foregoing circumstances, and an object of the present invention is to provide a method for calculating a residual stress, the method enabling a residual stress to be easily calculated without using a finite element method to determine a coefficient matrix representing a relation between an inherent strain and an elastic strain.

[MEANS FOR SOLVING THE PROBLEMS]

A first aspect of the present invention is defined in claim 1.

In the method as defined in claim 1, for each of the plurality of the finite element models each having the shape identical to that of the metal material after the cutting, the calculated value of the residual stress is derived based on the finite element analysis. Then, in the method for calculating a residual stress, the optimization problem of minimizing the difference between the calculated value and the measured value of the residual stress is solved, whereby the optimal solution of the parameter at which the inherent strain is applied is determined, and the residual stress in the finite element model having the shape identical to that of the metal material before the cutting is calculated using the parameter value at which the optimal solution is determined. Thus, according to the method for calculating a residual stress, a residual stress can be easily calculated without using a finite element method to determine a coefficient matrix representing a relation between an inherent strain and an elastic strain.

A second aspect of the present invention is defined in claim 2.

In the method as defined in claim 2, for each of the plurality of the finite element models each having the shape identical to that of the metal material after the cutting, the calculated value of the elastic strain is derived based on the finite element analysis. Then, in the method for calculating a residual stress, the optimization problem of minimizing the difference between the calculated value and the measured value of the elastic strain is solved, whereby the optimal solution of the parameter at which the inherent strain is applied is determined, and the residual stress in the finite element model having the shape identical to that of the metal material before the cutting is calculated using the parameter value at which the optimal solution is determined. Thus, according to the method for calculating a residual stress, a residual stress can be easily calculated without using a finite element method to determine a coefficient matrix representing a relation between an inherent strain and an elastic strain.

It is preferred that the metal material includes an axis portion having a cylindrical shape and a plate-shaped portion protruding radially from the entire circumference of the axis portion, and that the surface of the metal material includes a fillet face which is formed on a connection portion between the axis portion and the plate-shaped portion. "Protruding radially from the entire circumference of the axis portion" as referred to herein means that the plate-shaped portion protrudes radially from the entire outer periphery of the axis portion. In the fillet face which is formed on the connection portion between the axis portion and the plate-shaped portion, an inherent strain is likely to be generated during production. In the method for calculating a residual stress, a range including the fillet face is included in the calculation of the residual stress; therefore, the residual stress which is generated in the metal material can be appropriately evaluated.

It is preferred that the fillet face includes a region formed in an arc shape, and that an extended face of the cutting face passes through a center position of an arc of the fillet face. Thus, in the method for calculating a residual stress, the center position of the arc of the fillet face is defined as a center of a local coordinate system, whereby the finite element analysis can be efficiently performed.

In the setting, the parameter is preferably initialized based on at least one of a half-width of diffracted X-rays in X-ray stress measurement of the metal material, hardness of the metal material, a similar case, and the finite element analysis. The half-width of the diffracted X-rays in the X-ray stress measurement of the metal material, the hardness of the metal material, the similar case, and/or the finite element analysis enable(s) a plastic strain existing in the metal material to be estimated. Furthermore, in the optimization problem, the closer to the optimal solution an initial value of a design variable is, the more quickly the design variable converges to the optimal solution. In the method for calculating a residual stress, the estimation of the plastic strain enables a value close to the optimal solution to be selected as the initial value of the parameter, allowing the optimization problem to be efficiently solved.

An adjustable range of the parameter at a time of solving the optimization problem is preferably set under a constraint based on at least one of a half-width of diffracted X-rays in X-ray stress measurement of the metal material, hardness of the metal material, a similar case, and the finite element analysis. As such a rational constraint is employed for the parameter in the method for calculating a residual stress, the optimization problem can be efficiently solved.

In the method for calculating a residual stress, it is preferred that in the calculating, in a case in which the calculated value falls within an error range of the measured value, the difference between the calculated value and the measured value is defined as zero, and in a case in which the calculated value is outside of the error range of the measured value, the difference between the calculated value and the measured value is defined as a difference between the calculated value and a boundary of the error range. In the method for calculating a residual stress, the difference between the calculated value and the measured value is calculated based on the error range of the measured value, enabling a measurement error to be appropriately considered even in a case of an extremely large number of measurement points.

In the method for calculating a residual stress, as a constraint at a time of solving the optimization problem, a condition in which the metal material does not change in volume due to generation of the inherent strain is preferably employed. Thus, in the method for calculating a residual stress, a number of design variables in the optimization problem can be reduced.

The parameter is preferably the inherent strain or a coefficient of an inherent strain approximation function. Thus, the method for calculating a residual stress enables an optimal solution of the inherent strain to be directly calculated.

[EFFECTS OF THE INVENTION]

According to the method for calculating a residual stress of the present invention, a residual stress can be easily calculated without using a finite element method to determine a coefficient matrix representing a relation between an inherent strain and an elastic strain.

[BRIEF DESCRIPTION OF THE DRAWINGS]

Fig. 1 is a flow chart showing a method for calculating a residual stress of a first embodiment of the present invention.
Fig. 2 is a schematic longitudinal cross-sectional view of a metal material which is used in the method for calculating a residual stress in Fig. 1.
Fig. 3 is a schematic perspective cross-sectional view showing a part of the metal material in Fig. 2.
Fig. 4 is an explanatory diagram showing details of a procedure for determining an optimal solution in Fig. 1.
Fig. 5 is a graph showing a relation between an X-ray half-width and an equivalent inherent strain.
Fig. 6 is a graph showing a relation between a residual stress and a depth from a fillet face at $\theta = 30°$.

[DESCRIPTION OF EMBODIMENTS]

Hereinafter, embodiments of a method for calculating a residual stress of the present invention will be described in detail with reference to the drawings.

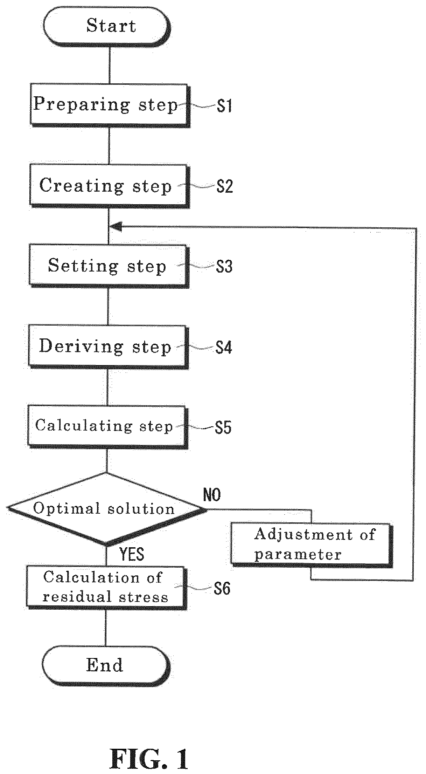

A method for calculating a residual stress shown in Fig. 1 is a method for calculating a residual stress in a metal material 1, the method using an inherent strain method and including: preparing a measured value of a residual stress in each of a plurality of portions on a surface of the metal material 1 and each of a plurality of portions on a cutting face which is formed by cutting the metal material 1 (preparing step S1); creating a plurality of finite element models each having a shape identical to that of the metal material 1 after the cutting, the metal material 1 after the cutting including a part of the surface of the metal material 1 and the cutting face (creating step S2); setting, for each of the plurality of the finite element models created in the creating step S2, a parameter enabling an inherent strain to be applied (setting step S3); deriving, for each of the plurality of the finite element models for which the parameter is set in the setting step S3, a calculated value of the residual stress in each of the plurality of the portions on the surface and each of the plurality of the portions on the cutting face, based on finite element analysis (deriving step S4); and calculating, for each of the plurality of the portions on the surface and each of the plurality of the portions on the cutting face, a difference between the calculated value of the residual stress derived in the deriving step S4 and the measured value of the residual stress prepared in the preparing step S1 (calculating step S5). In the method for calculating a residual stress, as an optimization problem of minimizing the difference between the calculated value calculated in the calculating step S5 and the measured value, after an optimal solution of the parameter is determined by repeating the setting step S3, the deriving step S4, and the calculating step S5 while adjusting the parameter, a parameter value at which the optimal solution is determined is set for a finite element model having a shape identical to that of the metal material 1 before the cutting, and a residual stress is calculated for the finite element model, based on the finite element analysis.

A principle of calculating a residual stress, a principle of calculating an inherent strain, and a metal material and an information processing device which are used in the method for calculating a residual stress will be described before specifically describing the method for calculating a residual stress.

Principle of Calculating Residual Stress

First, the principle of calculating a residual stress will be described. A residual stress $\sigma$ is represented by equation (1), wherein $\varepsilon_0$ denotes an inherent strain.

$$\sigma = D(\varepsilon - \varepsilon_0) \qquad \cdots (1)$$

In the equation (1), D denotes an elastic coefficient matrix, and $\varepsilon$ denotes a total strain that satisfies a relation in equation (2).

$$\int B^T \sigma \, dV = \int B^T D(\varepsilon - \varepsilon_0) \, dV = 0 \quad \cdots (2)$$

A range of a volume integral is the whole range of an object to be analyzed. Furthermore, B denotes a coefficient matrix that associates displacement u with $\varepsilon$, and a relation therebetween is represented by equation (3).

$$\varepsilon = Bu \qquad \cdots (3)$$

Meanwhile, a relation between a load and the displacement is represented by equation (4).

$$Ku = P \qquad \cdots (4)$$

In the equation (4), K denotes a stiffness matrix, and P denotes a load vector. According to a principle of virtual work, which is a basic theory of the finite element method, the stiffness matrix K is represented by equation (5).

$$K = \int B^T DB \, dV = 0 \qquad \cdots (5)$$

Assuming that the load is generated only by the inherent strain, the load vector P is represented by equation (6).

$$P = \int B^T D\varepsilon_0 \, dV = 0 \qquad \cdots (6)$$

When the inherent strain $\varepsilon_0$ is known, the displacement u can be determined using the equations (4) to (6). By substituting the displacement u thus determined into the equation (3) and by further using the equation (1), the total strain $\varepsilon$ and the residual stress $\sigma$ are obtained.

Principle of Calculating Inherent Strain

In a case in which $\sigma_m$ denotes N residual stresses determined by measurement and $\varepsilon_0$ denotes the inherent strain, $\sigma_c$, denotes N residual stresses to be calculated. A difference R between $\sigma_m$ and $\sigma_c$ is defined by equation (7).

$$R = (\sigma_m - \sigma_c)^T (\sigma_m - \sigma_c) \qquad \cdots (7)$$

The inherent strain can be approximately represented by a distribution function. The inherent strain $\varepsilon_0$ at a given point is represented by M distribution function parameters a as a linear function in equation (8).

$$\varepsilon_0 = Ma \qquad \cdots (8)$$

In the equation (8), M denotes a function of coordinates, wherein the function may be non-linear with respect to the coordinates. Assuming that the inherent strain $\varepsilon_0$ is represented by the equation (8), $\sigma_c$ can be determined using the equations (1) to (6). Accordingly, $\sigma_c$ can be represented by a linear function in equation (9).

$$\sigma_c = Ha \qquad \cdots (9)$$

In the equation (9), H denotes a coefficient matrix representing a relation between an inherent strain and an elastic strain.
In a general inherent strain method, the coefficient matrices M and H are determined by repeating a procedure of determining the residual stress, wherein in the procedure, for each component of the inherent strain $\varepsilon_0$, a unit value is applied to one component of the M distribution function parameters a, whereas the other components are assumed to be zero. Then, the equation (9) is substituted into the equation (7), and the M distribution function parameters a are determined such that R is minimized, whereby a most probable value of the inherent strain $\varepsilon_0$ is calculated. However, calculation of the coefficient matrix H representing the relation between the inherent strain and the elastic strain requires great time and effort.

Meanwhile, in the method for calculating a residual stress, the parameter enabling the inherent strain $\varepsilon_0$ to be applied is set for the finite element model, and for the finite element model for which the parameter is set, the calculated value of the residual stress is derived based on the finite element analysis. Then, in the method for calculating a residual stress, the optimization problem of minimizing the difference R between the calculated value and the measured value of the residual stress is solved using the equation (7), whereby the most probable value of the parameter is determined. In other words, in the method for calculating a residual stress, the most probable value of the inherent strain $\varepsilon_0$ is calculated without determining the coefficient matrix H representing the relation between the inherent strain and the elastic strain.

Metal Material

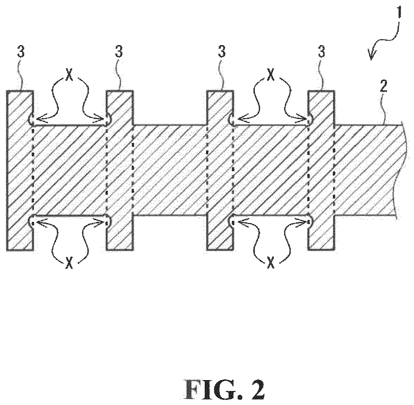

As shown in Fig. 2, the metal material 1 which is used in the method for calculating a residual stress includes an axis portion 2 having a cylindrical shape and plate-shaped portions 3 protruding radially from the entire circumference of the axis portion 2. Furthermore, fillet faces X each having a ring shape are formed on connection portions between the axis portion 2 and the plate-shaped portions 3 so as to circle around the axis portion 2. A plurality of portions on the fillet faces X are regions for which residual stresses are to be measured. In other words, the surface of the metal material 1 corresponding to the regions for which the residual stresses are to be measured includes the fillet faces X which are formed on the connection portions between the axis portion 2 and the plate-shaped portions 3. Examples of a material of the metal material 1 include metals such as forged steel, cast steel, titanium, and the like. It is to be noted that a number of the plate-shaped portions 3 is not limited to four as shown in Fig. 2.

Fillet Face

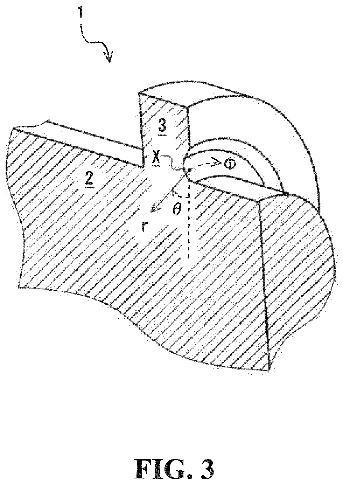

Each of the fillet faces X is formed in roughly an arc shape by cold working. Specifically, as shown in Fig. 3, in a longitudinal cross section (a divided face passing through a center line of the axis portion 2) of the metal material 1, each of the fillet faces X is formed so as to include a region formed in an arc shape (an arc-shaped region). It is to be noted that each of the fillet faces X needs only to be formed so as to include the arc-shaped region having a predetermined diameter (a predetermined fillet diameter), and may include a non-arc-shaped region in a part thereof.

Local Coordinate System

Here, a local coordinate system is defined to enable efficient performance of the finite element analysis described later. Since each of the fillet faces X includes the region formed in an arc shape and is formed in a ring shape so as to circle around the axis portion 2, each of the fillet faces X as a whole constitutes a part of a torus. Thus, a center position of the arc of each of the fillet faces X forms a circular orbit around the center line of the axis portion 2. In this context, in the longitudinal cross section of the metal material 1, the center position of the arc is defined as an origin of the local coordinate system, and a radius vector from the origin is defined as r. Furthermore, a direction of a perpendicular line from the center position of the arc of each of the fillet faces X toward the center line of the axis portion 2 is defined as a reference direction, and an argument from the reference direction in the longitudinal cross section of the metal material 1 is defined as $\theta$. Accordingly, a depth from each of the fillet faces X can be expressed using r, and a position on the arc of each of the fillet faces X can be expressed using $\theta$. Moreover, an argument with respect to a tangential direction of the circular orbit which is formed by the center position of the arc of each of the fillet faces X is defined as $\varphi$. By employing such a local coordinate system and assuming that a residual stress is uniformly generated in a circumferential direction of the axis portion 2, it becomes unnecessary to consider the argument $\varphi$, facilitating the finite element analysis in the vicinity of each of the fillet faces X.

Information Processing Device

In the method for calculating a residual stress, an information processing device which includes at least a control portion, a storage portion, a display portion, and an operation portion is used. The information processing device is, for example, a general-purpose computer, and, by having the control portion load a program stored in the storage portion, operates in such a way as to actualize the method for calculating a residual stress. Furthermore, the information processing device further includes an external information-acquiring portion for acquiring information from an external source and storing it in the storage portion. Examples of the external information-acquiring portion include a communication portion that transmits and receives information via a communication line or a communication wire which is connected to another device, a portable medium-reading portion that reads information from a portable medium storing the information, and the like.

Hereinafter, the method for calculating a residual stress will be specifically described.

Preparing Step S1

The preparing step S 1 is a step of preparing the measured value of the residual stress in each of the plurality of the portions on the surface of the metal material 1 and each of the plurality of the portions on the cutting face which is formed by cutting the metal material 1. In the preparing step S1, the information processing device imports measurement data of the residual stresses from the external information-acquiring portion and stores the measurement data in the storage portion. It is to be noted that in a case in which the information processing device is configured to serve also as a device for measuring the residual stresses, the communication portion receives the measurement data transmitted from a measurement apparatus, and the storage portion stores the measurement data.

Measurement Data

The measurement data is constituted by at least data associating measurement portions of the residual stresses with measured values of the residual stresses. The metal material 1 is a metallic structure in which the fillet faces X are formed by cold working; therefore, an inherent strain is generated in the vicinity of each of the fillet faces X. In other words, it is surmised that an elastic strain originating from the inherent strain is generated in a range from each of the fillet faces X to a certain depth. Therefore, the measurement portions of the residual stresses in the metal material 1 are selected to cover the range in which the elastic strain originating from the inherent strain is presumed to be generated. It is to be noted that the measurement data may further include data of measurement directions of the residual stresses and/or shape data of the metal material 1 after the cutting.

Furthermore, in the measurement of the residual stresses, to measure residual stresses in a variety of directions, the metal material 1 is cut so as to have a variety of cutting faces; in particular, the metal material 1 is cut with consideration given to the origin in the local coordinate system. Specifically, the metal material 1 is cut such that an extended face of each of the cutting faces of the metal material 1 passes through the center position of the arc of a corresponding fillet face of the fillet faces X. Thus, in the method for calculating a residual stress, the measurement portions of the residual stresses in the metal material 1 can be expressed using the radius vector r and the argument $\theta$, enabling the finite element analysis to be efficiently performed.

Creating Step S2

The creating step S2 is a step of creating the plurality of the finite element models each having the shape identical to that of the metal material 1 after the cutting, the metal material 1 after the cutting including the part of the surface of the metal material 1 and the cutting face. In the creating step S2, the information processing device creates, based on operation of the operation portion, the finite element models each having a shape identical to that of the metal material 1 at a time of measuring the residual stress. Commercially available finite element model-creating software is preferably used to create the finite element models.

In the measurement of the residual stresses, the metal material 1 is cut so as to have a variety of cutting faces; therefore, the plurality of the finite element models each having the shape identical to that of the metal material 1 after the cutting, the metal material 1 after the cutting including the part of the surface of the metal material 1 and the cutting face, are created in the creating step S2. It is to be noted that in a case in which the measurement data of the residual stresses includes the shape data of the metal material 1 after the cutting, the finite element models are preferably created based on the shape data.

Finite Element Model

The finite element models are formed by a plurality of elements each having specific identification information. A number of divisions of the finite element models is voluntarily set in accordance with a balance between computing capability of the information processing device and accuracy of calculating the residual stress. It is to be noted that in light of obtaining sufficient calculation accuracy, a division size of the finite element models in the range in which the elastic strain originating from the inherent strain is presumed to be generated is preferably smaller than a division size in other ranges. Furthermore, the finite element models are preferably divided in accordance with the radius vector r, the argument $\theta$, and the argument $\varphi$ in the local coordinate system. When the finite element models are thus divided, the finite element analysis is facilitated.

Setting Step S3

The setting step S3 is a step of setting, for each of the plurality of the finite element models created in the creating step S2, the parameter enabling the inherent strain to be applied. In the setting step S3, the information processing device sets, for each of the elements of the plurality of the finite element models, a parameter determined in advance.

Parameter

The parameter which is set for each of the elements is the parameter enabling the inherent strain to be applied to the finite element model. Examples of the parameter enabling the inherent strain to be applied include the inherent strain, a coefficient of an inherent strain approximation function, a temperature, energy, and the like. The parameter is preferably the inherent strain or the coefficient of the inherent strain approximation function because in such a case, the optimal solution of the inherent strain can be directly calculated in analysis of the optimization problem. In particular, when the coefficient of the inherent strain approximation function is selected as the parameter, unknowns of the inherent strain are aggregated into distribution function parameters by approximating the inherent strain by a distribution function; therefore, the residual stress can be derived with high accuracy even when a number of measured values of the residual stresses is small.

Furthermore, a half-width of diffracted X-rays in X-ray stress measurement of the metal material 1, hardness of the metal material 1, a similar case, and/or the finite element analysis enable(s) a plastic strain existing in the metal material 1 to be estimated. In the optimization problem, the closer to the optimal solution an initial value of a design variable is, the more quickly the design variable converges to the optimal solution. For this reason, the parameter enabling the inherent strain to be applied is preferably initialized based on at least one of the half-width of the diffracted X-rays in the X-ray stress measurement of the metal material 1, the hardness of the metal material 1, the similar case, and the finite element analysis. When the parameter is initialized based on the estimation of the plastic strain, the efficiency of solving the optimization problem is increased.

It is to be noted that values of the parameter initialized for each of the elements may all be identical, or may be partly or entirely different from each other. Furthermore, the parameter may be initialized to a given value, or does not necessarily need to be initialized based on the estimation of the plastic strain.

Deriving Step S4

The deriving step S4 is a step of deriving, for each of the plurality of the finite element models for which the parameter is set in the setting step S3, the calculated value of the residual stress in each of the plurality of the portions on the surface and each of the plurality of the portions on the cutting face, based on the finite element analysis. In the deriving step S4, the information processing device derives, for each of the plurality of the finite element models, the calculated value of the residual stress in each of the plurality of the portions on the surface and each of the plurality of the portions on the cutting face, which correspond to measurement portions of the residual stresses in the metal material 1, based on the finite element analysis. A known method may be used for the finite element analysis. Thus, the information processing device can execute the finite element analysis based on, for example, commercially available finite element analysis software.

Calculating Step S5

The calculating step S5 is a step of calculating, for each of the plurality of the portions on the surface and each of the plurality of the portions on the cutting face, the difference between the calculated value of the residual stress derived in the deriving step S4 and the measured value of the residual stress prepared in the preparing step S1. In the calculating step S5, the information processing device calculates, for each of the measurement portions of the residual stresses in the metal material 1, the difference between the calculated value of the residual stress which is derived using the finite element model and the measured value of the residual stress which is stored in the storage portion.

In the calculating step S5, the difference between the calculated value and the measured value is calculated based on an error range of the measured value. Specifically, in the calculation of the difference between the calculated value and the measured value, the information processing device executes the calculation as follows: in a case in which the calculated value of the residual stress falls within the error range of the measured value of the residual stress, the difference between the calculated value and the measured value is defined as zero, and in a case in which the calculated value is outside of the error range of the measured value, the difference between the calculated value and the measured value is defined as a difference between the calculated value and a boundary of the error range of the measured value. Thus, even in a case of an extremely large number of measurement points of the residual stresses, calculation results in which a measurement error is appropriately considered can be obtained. It is to be noted that as the error range of the measured value, for example, a standard deviation for each of the measurement portions is preferably used.

Optimization Problem

In the method for calculating a residual stress, as the optimization problem of minimizing the difference between the calculated value of the residual stress calculated in the calculating step S5 and the measured value of the residual stress, the optimal solution of the parameter is determined by repeating the setting step S3, the deriving step S4, and the calculating step S5 while adjusting the parameter. Specifically, the information processing device sets, as an objective function, minimization of the difference between the calculated value of the residual stress and the measured value of the residual stress, and, by setting the parameter as a configuration variable, repeatedly calculates the difference based on the finite element models while adjusting the parameter, thereby determining an optimal solution of the parameter at which the difference is minimized. To search for the optimal solution, for example, a known method such as a Hooke-Jeeves method, which is a discrete search method, or the like is used. Thus, the information processing device is able to analyze the optimization problem based on commercially available optimization software.

It is to be noted that as the objective function, for example, a sum of squares of differences between the calculated value of the residual stress and the measured value of the residual stress may be used, or a maximum value of the difference between the calculated value of the residual stress and the measured value of the residual stress may be used. Furthermore, whether the optimal solution of the parameter has been obtained may be judged, for example, by judging, in the analysis of the optimization problem, whether a certain period of time has elapsed without a large change in the difference; in a case in which the certain period of time has elapsed, it may be determined that the optimal solution of the parameter has been obtained.

An adjustable range of the parameter at a time of solving the optimization problem is preferably set under a constraint based on at least one of the half-width of the diffracted X-rays in the X-ray stress measurement of the metal material 1, the hardness of the metal material 1, the similar case, and the finite element analysis. For example, in a case in which there are similar examination cases and variation of an inherent strain distribution is known, the adjustable range of the parameter may be set under a constraint based on a range of the variation. Furthermore, for example, a maximum value and a minimum value of the inherent strain may be predicted based on at least one of a result of reproducing, by the finite element analysis, a process of generating the residual stress in the metal material 1; a measurement result of the hardness of the metal material 1; and a measurement result of the half-width of the diffracted X-rays in the X-ray stress measurement of the metal material 1, and the adjustable range of the parameter may be set under a constraint corresponding to a result of the prediction. Furthermore, at least one of a maximum value and a minimum value of the adjustable range of the parameter is preferably set under a constraint. When a rational constraint is applied to the parameter, the efficiency of solving the optimization problem is increased.

Furthermore, in the method for calculating a residual stress, as a constraint at the time of solving the optimization problem, a condition in which the metal material 1 does not change in volume due to generation of the inherent strain is preferably employed. In a case in which the residual stress in the metal material 1 is generated by a plastic strain, it can be deemed that the metal material 1 does not change in volume, although a shape of a plastically deformed part is changed. Thus, by employing the constraint in the method for calculating a residual stress, a number of design variables in the optimization problem can be reduced.

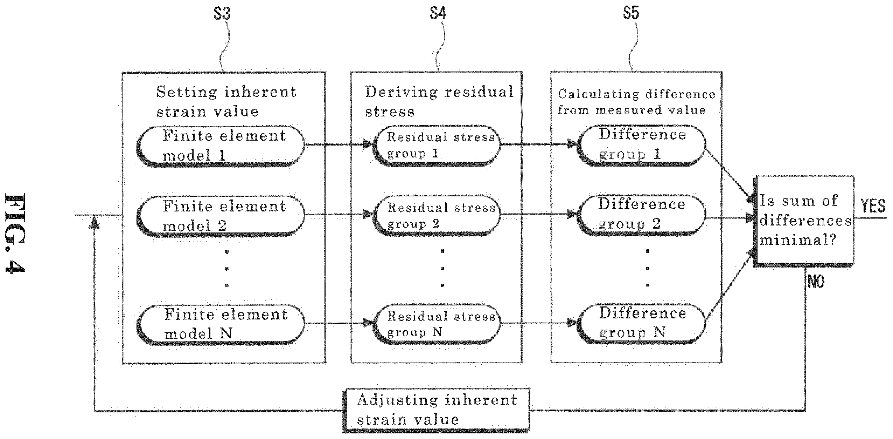

In the analysis of the optimization problem, N finite element models (N is a natural number of greater than or equal to 2) are used as shown in Fig. 4. As set forth above, first, in the setting step S3, for example, an inherent strain value is set as a parameter for each of the N finite element models. Next, in the deriving step S4, for each of the N finite element models, the calculated value of the residual stress in each of the plurality of the portions on the surface and each of the plurality of the portions on the cutting face which correspond to the measurement portions of the residual stresses in the metal material 1 is derived based on the finite element analysis. Since the residual stress is calculated for each of the plurality of the portions on the surface and each of the plurality of the portion on the cutting face, calculated values of the residual stresses are associated with each other as a residual stress group for each of the finite element models. Next, in the calculating step S5, for each of the plurality of the portions on the surface and each of the plurality of the portions on the cutting face, the difference between the calculated value and the measured value of the residual stress is calculated. Differences thus calculated are associated with each other as a difference group for each of the finite element models. Next, it is judged whether a sum of all the differences calculated is minimal. In a case in which the sum of the differences is not minimal, or in a case in which the calculating step S5 is performed for the first time, inherent strain values are adjusted based on the search method, and then the setting step S3 is returned to in order to reset, for the N finite element models, the inherent strain values thus adjusted. Then, this processing is repeated until the sum of the differences is minimized.

Calculating Residual Stress S6

In the method for calculating a residual stress, after the optimal solution of the parameter is determined by solving the optimization problem, the parameter value at which the optimal solution is determined is set for the finite element model having the shape identical to that of the metal material 1 before the cutting, and the residual stress is calculated for the finite element model based on the finite element analysis. Specifically, after determining the optimal solution of the parameter by solving the optimization problem described above, the information processing device sets, for the finite element model having the shape identical to that of the metal material 1 before the cutting, the parameter value at which the optimal solution is determined. Then, the information processing device calculates, for the finite element model, a residual stress in a given position, based on the finite element analysis. In this manner, according to the method for calculating a residual stress, an estimated value of the residual stress in the given position of the metal material 1 can be calculated.

Advantages

In the method for calculating a residual stress, for each of the plurality of the finite element models each having the shape identical to that of the metal material 1 after the cutting, the calculated value of the residual stress is derived based on the finite element analysis. Then, in the method for calculating a residual stress, the optimization problem of minimizing the difference between the calculated value and the measured value of the residual stress is solved, whereby the optimal solution of the parameter at which the inherent strain is applied is determined, and the residual stress in the finite element model having the shape identical to that of the metal material 1 before the cutting is calculated using the parameter value at which the optimal solution is determined. Thus, according to the method for calculating a residual stress, a residual stress can be easily calculated without using a finite element method to determine a coefficient matrix representing a relation between an inherent strain and an elastic strain.

In the method for calculating a residual stress, a range including the fillet face X of the metal material 1 is included in the calculation of the residual stress; therefore, the residual stress which is generated in the metal material 1 can be appropriately evaluated. Furthermore, the fillet face X includes the region formed in an arc shape, and the extended face of the cutting face of the metal material 1 passes through the center position of the arc of the fillet face X; therefore, according to the method for calculating a residual stress, by defining the center position of the arc of the fillet face X as the center of the local coordinate system, the finite element analysis can be efficiently performed.

In the calculating step S5 of the method for calculating a residual stress, in the case in which the calculated value of the residual stress falls within the error range of the measured value, the difference between the calculated value and the measured value is defined as zero, and in the case in which the calculated value is outside of the error range of the measured value, the difference between the calculated value and the measured value is defined as the difference between the calculated value and the boundary of the error range; therefore, even in a case of an extremely large number of measurement points, a measurement error can be appropriately considered.

Other Embodiments

It should be understood that the embodiments disclosed herein are illustrative and non-restrictive in every respect. The scope of the present invention is not limited to the structures of the above embodiments, is defined by the terms of the claims, and is intended to include any modifications within the scope and meaning equivalent to the terms of the claims.

In the above embodiment, for each of the plurality of the finite element models each having the shape identical to that of the metal material 1 after the cutting, the calculated value of the residual stress is derived based on the finite element analysis, and the optimization problem of minimizing the difference between the calculated value and the measured value of the residual stress is solved, whereby the optimal solution of the parameter at which the inherent strain is applied is determined; however, the following method may also be employed: for each of the plurality of the finite element models each having the shape identical to that of the metal material 1 after the cutting, a calculated value of an elastic strain is derived based on the finite element analysis, and an optimization problem of minimizing a difference between the calculated value and a measured value of the elastic strain is solved, whereby the optimal solution of the parameter at which an inherent strain is applied is determined. In other words, the method for calculating a residual stress may be a method for calculating a residual stress in the metal material 1, the method using an inherent strain method and including: preparing a measured value of an elastic strain in each of a plurality of portions on a surface of the metal material 1 and each of a plurality of portions on a cutting face which is formed by cutting the metal material 1; creating a plurality of finite element models each having a shape identical to that of the metal material 1 after the cutting, the metal material 1 after the cutting including a part of the surface of the metal material 1 and the cutting face; setting, for each of the plurality of the finite element models created in the creating, a parameter enabling an inherent strain to be applied; deriving, for each of the plurality of the finite element models for which the parameter is set in the setting, a calculated value of the elastic strain in each of the plurality of the portions on the surface and each of the plurality of the portions on the cutting face, based on finite element analysis; and calculating, for each of the plurality of the portions on the surface and each of the plurality of the portions on the cutting face, a difference between the calculated value of the elastic strain derived in the deriving and the measured value of the elastic strain prepared in the preparing, wherein as an optimization problem of minimizing the difference between the calculated value calculated in the calculating and the measured value, after an optimal solution of the parameter is determined by repeating the setting, the deriving, and the calculating while adjusting the parameter, a parameter value at which the optimal solution is determined is set for a finite element model having a shape identical to that of the metal material 1 before the cutting, and a residual stress is calculated for the finite element model, based on the finite element analysis.

In the above embodiment, the metal material 1 includes the axis portion 2 having a cylindrical shape and the four plate-shaped portions 3 protruding radially from the entire circumference of the axis portion 2, and has the fillet faces X on the connection portions between the axis portion 2 and the plate-shaped portions 3; however, the metal material which is used in the method for calculating a residual stress is not limited to a metal material having such a structure. For example, the metal material does not necessarily need to have the fillet faces.

In the above embodiment, each of the fillet faces X of the metal material 1 includes the region formed in an arc shape, and at the time of measuring the residual stress in the metal material 1, the metal material 1 is cut such that the extended face of the cutting face of the metal material 1 passes through the center position of the arc of the fillet face X; however, a structure may be employed in which the extended face of the cutting face of the metal material 1 does not pass through the center position of the arc of the fillet face X. It is to be noted that in light of the efficiency of the finite element analysis, the metal material 1 is preferably cut such that the extended face of the cutting face of the metal material 1 passes through the center position of the arc of the fillet face X.

In the above embodiment, the calculation in the calculating step S5 is executed as follows: in the case where the calculated value of the residual stress falls within the error range of the measured value of the residual stress, the difference between the calculated value and the measured value is defined as zero, and in the case in which the calculated value is outside of the error range of the measured value, the difference between the calculated value and the measured value is defined as the difference between the calculated value and the boundary of the error range of the measured value; however, the difference between the calculated value and the measured value may be calculated without considering a measurement error. It is to be noted that in light of obtaining a calculation result in which the measurement error is appropriately considered, the difference between the calculated value and the measured value is preferably calculated based on the error range of the measured value.

In the above embodiment, the preparing step S1 is performed before the creating step S2; however, the preparing step S1 may be performed at any time before the calculating step S5.

[EXAMPLES]

Hereinafter, the present invention will be described further in detail by way of Examples; however, the Examples should not be construed as limiting the present invention.

Measurement of Residual Stress

Regarding the metal material shown in Fig. 2, residual stresses on and inside the fillet faces were measured. Steel was used as a material of the metal material. The four fillet faces had been subjected to cold working in a similar manner, and each of the fillet faces included a region formed in an arc shape having a diameter of 22 mm. For simplicity, it is assumed that the four fillet faces were identical, and that residual stresses were uniformly generated in a circumferential direction of the axis portion. Furthermore, a center position of the arc having a diameter of 22 mm is defined as an origin of a local coordinate system.

In order for all residual stresses in three directions to be measurable, cutting pieces having three types of shapes, namely an F piece, a C piece, and a T piece, were prepared from the metal material. The F piece, the C piece, and the T piece were each formed such that an extended face of a cutting face passed through the origin of the local coordinate system. The F piece had a shape in which the axis portion was cut from a plate-shaped portion side at an angle of $\theta = 0°$, wherein residual stresses in a $\theta$ direction and a $\varphi$ direction in the vicinity of a surface of the fillet face could be measured while changing $\theta$. The C piece had a shape in which the plate-shaped portion was cut into a conical shape or the like from an axis portion side at an angle ranging from $\theta = 0°$ to $\theta = 110°$, wherein residual stresses in an r direction and the $\varphi$ direction inside the fillet face could be measured while changing $\theta$. The T piece had a thin shape which was obtained by slicing the metal material with $\varphi$ slightly changed so as to pass through a center line of the axis portion, wherein residual stresses in the r direction and the $\theta$ direction inside the fillet face could be measured while changing $\theta$. One of each of the F piece and the T piece were prepared, and two C pieces, namely a piece for measuring while changing θ by increments of 20° from 0° to 100° (a Ca piece), and a piece for measuring while changing θ by increments of 20° from 10° to 110° (a Cb piece), were prepared. It is to be noted that measurement positions of the residual stresses were subjected to an electrolytic polishing treatment.

Regarding the F piece, residual stresses in positions at depths of 0.3 mm and 0.5 mm from the fillet surface were measured using an X-ray stress measurement apparatus ("MSF-3M," available from Rigaku Corporation). A $\sin^2\psi$ method was used for the measurement of the residual stresses. A θ-direction stress was measured while changing θ by increments of 10° in a range from 80° to 100°, and a φ-direction stress was measured while changing θ by increments of 10° in a range from 0° to 100°; thus, measured values at 28 points in total were obtained.

Regarding the two C pieces, residual stresses in positions at depths of 18 mm to 40 mm from the fillet surface were measured using the X-ray stress measurement apparatus ("MSF-3M," available from Rigaku Corporation). The $\sin^2\psi$ method was used for the measurement of the residual stresses. For the Ca piece, an r-direction stress and a φ-direction stress were each measured at 10 to 16 points while changing θ by increments of 20° in a range from 0° to 100° by cutting, and for the Cb piece, an r-direction stress and a φ-direction stress were each measured at 10 to 16 points while changing θ by increments of 20° in a range from 10° to 110° by cutting; thus, measured values at 308 points in total were obtained.

Regarding the T piece, residual stresses in positions at depths of 18 mm to 40 mm from the fillet surface were measured using an X-ray residual stress measurement apparatus ("μ-360," available from Pulstec Industrial Co., Ltd.). A cos α method was used for the measurement of the residual stresses. An r-direction stress and a θ-direction stress were each measured at 10 to 16 points while changing θ by increments of 10° in a range from 0° to 100°; thus, measured values at 222 points in total were obtained.

Preparation of Finite Element Model

In an information processing device, finite element models having shapes identical to those of the F piece, the C piece, and the T piece, respectively, were created using finite element method-analysis software. It is to be noted that for the C piece, since θ had been changed by the cutting, 12 types of finite element models each having a shape identical to that at a time of measuring the residual stress were created.

To employ a method in which an inherent strain is approximated by a distribution function, for each of the finite element models thus created, an inherent strain was set using a distribution function in equations (10) and (11).

$$P(\xi, \omega) = \sum_{i=1}^{m} \sum_{j=1}^{n} A_{ij} \, (1 - \xi)^i \sin(j\pi\omega) \quad \cdot \cdot \cdot (1\ 0)$$

$$\xi = \frac{R - R_0}{\Delta R}, \omega = \frac{\alpha - \alpha_0}{\Delta \alpha} + \frac{1}{2} \quad \cdot \cdot \cdot (1\ 1)$$

In the equation (11), R denotes the r direction, and α denotes the θ direction. $R_0$ and $\alpha_0$ denote starting positions for calculation of the inherent strain, and ΔR and Δα denote incremental values. Furthermore, in the equation (10), m and n denote orders in the r direction and the θ direction, respectively, and $A_{ij}$ denotes a coefficient of an approximation function. $R_0$, $\alpha_0$, ΔR, and Δα are parameters which determine an inherent strain-calculation region; even when identical residual stress data is used, a difference in setting values of these parameters results in a difference in an inherent strain distribution to be obtained. Similarly, the inherent strain distribution to be obtained also varies depending on values of the orders m and n of the distribution function of the inherent strain.

Since the metal material shown in Fig. 2 is axisymmetric, a shear strain in the φ direction is 0. Therefore, it is only necessary to consider strains of four components. Coefficients $A_{ij}$ which were used to calculate the strains of these components were denoted by $a_{ij}$, $b_{ij}$, $c_{ij}$, and $d_{ij}$, respectively.

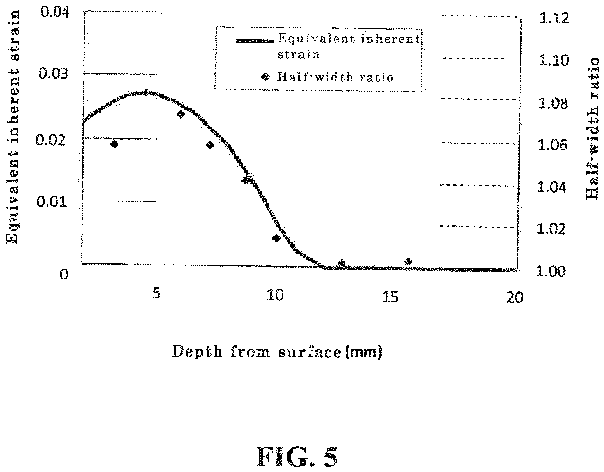

As shown in Fig. 5, it is known that an equivalent inherent strain has a correlation with a half-width (an X-ray half-width) in X-ray residual stress measurement. Furthermore, assuming that the inherent strain is generated only by a plastic strain, the inherent strain has a correlation with the X-ray half-width. Thus, initial values of the coefficients of the approximation function of the inherent strain were determined based on a measurement result of the X-ray half-width. When m is 2 and n is 8, $a_{ij}$, $b_{ij}$, $c_{ij}$, and $d_{ij}$ are each 16 coefficients. Table 1 shows the initial values of the coefficients of the approximation function, wherein the initial values were determined based on the measurement result of the X-ray half-width under the condition.

Table 1

| j= | 1 | 2 | 3 | 4 | 5 | 6 | 7 | 8 |
|---|---|---|---|---|---|---|---|---|
| $a_{1j}$ | -0.02 | -0.01 | 0.02 | 0.01 | 0 | 0.01 | 0.02 | 0.01 |
| $a_{2j}$ | 0.04 | 0.01 | -0.02 | -0.01 | -0.01 | 0 | -0.02 | -0.02 |
| $b_{1j}$ | 0.01 | 0 | -0.01 | 0 | 0.03 | 0.02 | 0 | 0 |
| $b_{2j}$ | 0.02 | -0.02 | -0.01 | 0.01 | -0.02 | -0.03 | 0 | 0.01 |
| $c_{1j}$ | -0.02 | 0 | 0.01 | -0.01 | 0.01 | 0.02 | 0.01 | 0 |
| $c_{2j}$ | 0.07 | 0.02 | -0.01 | 0 | 0.02 | 0 | -0.03 | 0 |
| $d_{1j}$ | 0.01 | 0.01 | 0.01 | 0.01 | 0.01 | 0.01 | 0.01 | 0.01 |
| $d_{2j}$ | 0.01 | 0.01 | 0.01 | 0 | 0 | 0 | -0.01 | -0.01 |

Analysis of Optimization Problem

An optimization problem was analyzed, wherein design variables were the coefficients $a_{ij}$, $b_{ij}$, $c_{ij}$, and $d_{ij}$ of the approximation function of the inherent strain, and wherein an objective function was to minimize a difference between a measured value and a calculated value of the residual stress. As a constraint in the optimization problem, absolute values of the coefficients $a_{ij}$, $b_{ij}$, $c_{ij}$, and $d_{ij}$ were set to be less than 0.1. Furthermore, general-purpose analysis software ("Isight," available from Dassault Systemes) was used for optimization calculation, and a Hooke-Jeeves method was employed for optimization search.

Firstly, residual stresses in positions corresponding to the positions at which the residual stresses were actually measured were calculated by finite element analysis using the finite element models for which the inherent strain was set using the distribution function. Next, for each of the finite element models, a square of the difference between the measured value and the calculated value of the residual stress was calculated for each of the positions, and resulting squares were added together. Then, the foregoing calculation was repeated while adjusting the values of the coefficients $a_{ij}$, $b_{ij}$, $c_{ij}$, and $d_{ij}$ by the Hooke-Jeeves method such that a sum of the squares of differences was minimized. At a point of time at which, in calculation over a certain period of time, there was no large change in the sum of the squares of the differences, it was determined that the sum of the squares of the differences was minimized.

Calculation of Residual Stress

For a finite element model having a shape identical to that of the metal material in Fig. 2 before the cutting, optimal solutions of the coefficients $a_{ij}$, $b_{ij}$, $c_{ij}$, and $d_{ij}$ were set, and a residual stress at $\theta = 30°$ with respect to the depth was calculated for the finite element model based on the finite element analysis. Furthermore, for comparison, a residual stress in the metal material in Fig. 2 at $\theta = 30°$ with respect to the depth was measured by a cutting method using a strain gauge. A measurement direction of the residual stress was the $\theta$ direction. In the cutting method, the residual stress was ascertained as follows: a hole was drilled such that a measurement portion was exposed, the strain gauge was attached to a bottom portion of the hole, the metal material was cut into a smallest piece such that the smallest piece included the portion to which the strain gauge was attached, and released elastic strains before and after the cutting were measured.

Furthermore, in the calculation of the difference between the measured value and the calculated value of the residual stress, calculation considering an error range was also examined as follows: in a case in which the calculated value fell within the error range of the measured value, the difference between the calculated value and the measured value was defined as zero, and in a case in which the calculated value was outside of the error range of the measured value, the difference between the calculated value and the measured value was defined as a difference between the calculated value and a boundary of the error range. As the error range of the measured value, a standard deviation for each of the measurement portions was used.

Moreover, an analysis method was also examined in which as a constraint at a time of solving the optimization problem, a condition in which the metal material did not change in volume due to generation of the inherent strain. The condition in which the volume did not change resulted in a sum of $a_{ij}$, $b_{ij}$, and $c_{ij}$ being 0, enabling a reduction in the number of design variables.

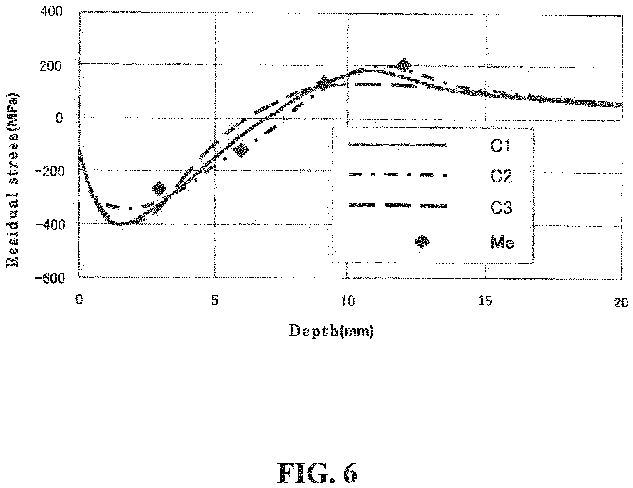

Fig. 6 shows each of a result (C1) of calculating the residual stress without considering the error range; a result (C2) of calculating the residual stress, considering the error range; a result (C3) of calculating the residual stress, considering the constraint in which the volume does not change; and a result (Me) of measuring the residual stress by the cutting method.

As shown in Fig. 6, it was confirmed that all the results of calculating the residual stress were highly consistent with the result of measuring the residual stress by the cutting method. In particular, it can be said that the result of calculating the residual stress, considering the error range, is most consistent with the result of measuring the residual stress. Furthermore, it can be said that despite the smaller number of design variables, the result of calculating the residual stress, considering the constraint in which the volume does not change, was sufficiently consistent with the result of measuring the residual stress.

[INDUSTRIAL APPLICABILITY]

According to the method for calculating a residual stress of the present invention, a residual stress can be easily calculated without using a finite element method to determine a coefficient matrix representing a relation between an inherent strain and an elastic strain.

[EXPLANATION OF THE REFERENCE SYMBOLS]

    1: metal material
    2: axis portion
    3: plate-shaped portion
    X: fillet face

**Claims**

1. A method for calculating a residual stress in a metal material (1), the method using an inherent strain method and comprising:

    preparing (S 1) a measured value of a residual stress in each of a plurality of portions on a surface of a metal material (1) and each of a plurality of portions on a cutting face which is formed by cutting the metal material (1);
    creating (S2) a plurality of finite element models each having a shape identical to that of the metal material (1) after the cutting, the metal material (1) after the cutting including a part of the surface and the cutting face;
    setting (S3), for each of the plurality of the finite element models created in the creating (S2), a parameter enabling an inherent strain to be applied;
    deriving (S4), for each of the plurality of the finite element models for which the parameter is set in the setting (S3), a calculated value of the residual stress in each of the plurality of the portions on the surface and each of the plurality of the portions on the cutting face, based on finite element analysis; and
    calculating (S5), for each of the plurality of the portions on the surface and each of the plurality of the portions on the cutting face, a difference between the calculated value of the residual stress derived in the deriving (S4) and the measured value of the residual stress prepared in the preparing (S 1), wherein
    as an optimization problem of minimizing the difference between the calculated value calculated in the calculating (S5) and the measured value, after an optimal solution of the parameter is determined by repeating the setting (S3), the deriving (S4), and the calculating (S5) while adjusting the parameter, a parameter value at which the optimal solution is determined is set for a finite element model having a shape identical to that of the metal material (1) before the cutting, and a residual stress is calculated for the finite element model, based on the finite element analysis.

2. A method for calculating a residual stress in a metal material (1), the method using an inherent strain method and comprising:

    preparing (S 1) a measured value of an elastic strain in each of a plurality of portions on a surface of a metal material (1) and a plurality of portions on a cutting face which is formed by cutting the metal material (1);
    creating (S2) a plurality of finite element models each having a shape identical to that of the metal material (1) after the cutting, the metal material (1) after the cutting including a part of the surface and the cutting face;
    setting (S3), for each of the plurality of the finite element models created in the creating (S2), a parameter enabling an inherent strain to be applied;

deriving (S4), for each of the plurality of the finite element models for which the parameter is set in the setting (S3), a calculated value of the elastic strain in each of the plurality of the portions on the surface and each of the plurality of the portions on the cutting face, based on finite element analysis; and

calculating (S5), for each of the plurality of the portions on the surface and each of the plurality of the portions on the cutting face, a difference between the calculated value of the elastic strain derived in the deriving (S4) and the measured value of the elastic strain prepared in the preparing (S1), wherein

as an optimization problem of minimizing the difference between the calculated value calculated in the calculating (S5) and the measured value, after an optimal solution of the parameter is determined by repeating the setting (S3), the deriving (S4), and the calculating (S5) while adjusting the parameter, a parameter value at which the optimal solution is determined is set for a finite element model having a shape identical to that of the metal material (1) before the cutting, and a residual stress is calculated for the finite element model, based on the finite element analysis.

3. The method for calculating a residual stress according to claim 1, wherein

the metal material (1) comprises an axis portion having a cylindrical shape and a plate-shaped portion protruding radially from the entire circumference of the axis portion, and
the surface of the metal material (1) comprises a fillet face which is formed on a connection portion between the axis portion and the plate-shaped portion.

4. The method for calculating a residual stress according to claim 2, wherein

the metal material (1) comprises an axis portion having a cylindrical shape and a plate-shaped portion protruding radially from the entire circumference of the axis portion, and
the surface of the metal material (1) comprises a fillet face which is formed on a connection portion between the axis portion and the plate-shaped portion.

5. The method for calculating a residual stress according to claim 3 or 4, wherein

the fillet face comprises a region formed in an arc shape, and
an extended face of the cutting face passes through a center position of an arc of the fillet face.

6. The method for calculating a residual stress according to any one of claims 1 to 4, wherein in the setting (S3), the parameter is initialized based on at least one of a half-width of diffracted X-rays in X-ray stress measurement of the metal material (1), hardness of the metal material (1), a similar case, and the finite element analysis.

7. The method for calculating a residual stress according to any one of claims 1 to 4, wherein an adjustable range of the parameter at a time of solving the optimization problem is set under a constraint based on at least one of a half-width of diffracted X-rays in X-ray stress measurement of the metal material (1), hardness of the metal material (1), a similar case, and the finite element analysis.

8. The method for calculating a residual stress according to any one of claims 1 to 4, wherein in the calculating (S5), in a case in which the calculated value falls within an error range of the measured value, the difference between the calculated value and the measured value is defined as zero, and in a case in which the calculated value is outside of the error range of the measured value, the difference between the calculated value and the measured value is defined as a difference between the calculated value and a boundary of the error range.

9. The method for calculating a residual stress according to any one of claims 1 to 4, wherein as a constraint at a time of solving the optimization problem, a condition in which the metal material (1) does not change in volume due to generation of the inherent strain is employed.

10. The method for calculating a residual stress according to any one of claims 1 to 4, wherein the parameter is the inherent strain or a coefficient of an inherent strain approximation function.

**Patentansprüche**

1. Verfahren zur Berechnung einer Restspannung in einem Metallwerkstoff (1), wobei das Verfahren ein Eigenspannungsverfahren verwendet und umfasst:

Bereitstellen (S1) eines Messwerts einer Restspannung in jedem einer Vielzahl von Abschnitten an einer Oberfläche eines Metallwerkstoffs (1) und jedem einer Vielzahl von Abschnitten an einer Schnittfläche, die durch Schneiden des Metallwerkstoffs (1) gebildet wird;

Erzeugen (S2) einer Vielzahl von Finite-Elemente-Modellen, die jeweils eine Form aufweisen, die mit jener des Metallwerkstoffs (1) nach dem Schneiden identisch ist, wobei der Metallwerkstoff (1) nach dem Schneiden einen Teil der Oberfläche und der Schnittfläche aufweist;

Festlegen (S3) eines Parameters, der das Aufbringen einer Eigenspannung ermöglicht, für jedes der Vielzahl von Finite-Elemente-Modellen, die bei dem Erzeugen (S2) erzeugt wurden;

Ableiten (S4) eines Berechnungswerts der Restspannung für jedes der Vielzahl von Finite-Elemente-Modellen, für die der Parameter bei dem Festlegen (3) festgelegt wurde, in jedem der Vielzahl von Teilen an der Oberfläche und jedem der Vielzahl von Teilen an der Schnittfläche auf Grundlage einer Finite-Elemente-Analyse; und

Berechnen (S5) einer Differenz zwischen dem berechneten Wert der beim Ableiten (S4) abgeleiteten Restspannung und dem Messwert der Restspannung, der bei dem Bereitstellen (S1) bereitgestellt wurde, für jeden der Vielzahl von Teilen an der Oberfläche und jeden der Vielzahl von Teilen an der Schnittfläche, wobei

als ein Optimierungsproblem des Minimierens der Differenz zwischen dem bei dem Berechnen (S5) berechneten Berechnungswert und dem Messwert, nachdem durch Wiederholen des Festlegens (S3), des Ableitens (S4) und des Berechnens (S5) unter gleichzeitiger Anpassung des Parameters eine Optimallösung des Parameters ermittelt wurde, ein Parameterwert, bei dem die Optimallösung ermittelt wird, für ein Finite-Elemente-Modell festgelegt wird, dessen Form identisch mit jener des Metallwerkstoffs (1) vor dem Schneiden ist, und eine Restspannung für das Finite-Elemente-Modell basierend auf der Finite-Elemente-Analyse berechnet wird.

2. Verfahren zur Berechnung einer Restspannung in einem Metallwerkstoff (1), wobei das Verfahren ein Eigenspannungsverfahren verwendet und umfasst:

Bereitstellen (S1) eines Messwerts einer elastischen Spannung in jedem einer Vielzahl von Abschnitten an einer Oberfläche eines Metallwerkstoffs (1) und einer Vielzahl von Abschnitten an einer Schnittfläche, die durch Schneiden des Metallwerkstoffs (1) gebildet wird,

Erzeugen (S2) einer Vielzahl von Finite-Elemente-Modellen, die jeweils eine Form aufweisen, die mit jener des Metallwerkstoffs (1) nach dem Schneiden identisch ist, wobei der Metallwerkstoff (1) nach dem Schneiden einen Teil der Oberfläche und der Schnittfläche aufweist;

Festlegen (S3) eines Parameters, der das Aufbringen einer Eigenspannung ermöglicht, für jedes der Vielzahl von Finite-Elemente-Modellen, die bei dem Erzeugen (S2) erzeugt wurden;

Ableiten (S4) eines Berechnungswerts der elastischen Spannung für jedes der Vielzahl von Finite-Elemente-Modellen, für die bei dem Festlegen (3) der Parameter festgelegt wurde, in jedem der Vielzahl von Teilen an der Oberfläche und jedem der Vielzahl von Teilen an der Schnittfläche auf Grundlage einer Finite-Elemente-Analyse; und

Berechnen (S5) einer Differenz zwischen dem berechneten Wert der bei dem Ableiten (S4) abgeleiteten elastischen Spannung und dem Messwert der elastischen Spannung, der bei dem Bereitstellen (S1) bereitgestellt wurde, für jeden der Vielzahl von Teilen an der Oberfläche und jeden der Vielzahl von Teilen an der Schnittfläche, wobei

als ein Optimierungsproblem des Minimierens der Differenz zwischen dem bei dem Berechnen (S5) berechneten Berechnungswert und dem Messwert, nachdem durch Wiederholen des Festlegens (S3), des Ableitens (S4) und des Berechnens (S5) unter gleichzeitiger Anpassung des Parameters eine Optimallösung des Parameters ermittelt wurde, ein Parameterwert, bei dem die Optimallösung ermittelt wird, für ein Finite-Elemente-Modell festgelegt wird, dessen Form identisch mit jener des Metallwerkstoffs (1) vor dem Schneiden ist, und eine Restspannung für das Finite-Elemente-Modell basierend auf der Finite-Elemente-Analyse berechnet wird.

3. Verfahren zur Berechnung einer Restspannung nach Anspruch 1, wobei

der Metallwerkstoff (1) einen Achsabschnitt mit zylindrischer Form und einen plattenförmigen Abschnitt, der vom Gesamtumfang des Achsabschnitts radial vorsteht, aufweist, und

die Oberfläche des Metallwerkstoffs (1) eine Kehlfläche aufweist, die an einem Verbindungsabschnitt zwischen dem Achsabschnitt und dem plattenförmigen Abschnitt gebildet ist.

4. Verfahren zur Berechnung einer Restspannung nach Anspruch 2, wobei

der Metallwerkstoff (1) einen Achsabschnitt mit zylindrischer Form und einen plattenförmigen Abschnitt, der vom Gesamtumfang des Achsabschnitts radial vorsteht, aufweist, und

die Oberfläche des Metallwerkstoffs (1) eine Kehlfläche aufweist, die an einem Verbindungsabschnitt zwischen dem Achsabschnitt und dem plattenförmigen Abschnitt gebildet ist.

**5.** Verfahren zur Berechnung einer Restspannung nach Anspruch 3 oder 4, wobei

die Kehlfläche einen bogenförmig ausgebildeten Bereich aufweist, und
eine Verlängerungsfläche der Schnittfläche durch eine Mittenposition eines Bogens der Kehlfläche verläuft.

**6.** Verfahren zur Berechnung einer Restspannung nach einem der Ansprüche 1 bis 4, wobei bei dem Festlegen (S3) der Parameter auf Grundlage einer Halbwertsbreite gebeugter Röntgenstrahlung bei einer Röntgenstrahlbelastungsmessung des Metallwerkstoffs (1) und/oder der Härte des Metallwerkstoffs (1) und/oder einem ähnlichen Fall und/oder der Finite-Elemente-Analyse initialisiert wird.

**7.** Verfahren zur Berechnung einer Restspannung nach einem der Ansprüche 1 bis 4, wobei ein einstellbarer Bereich des Parameters zum Zweipunkt des Lösens des Optimierungsproblems unter einer Beschränkung basierend auf einer Halbwertsbreite gebeugter Röntgenstrahlung bei einer Röntgenstrahlbelastungsmessung des Metallwerkstoffs (1) und/oder einer Härte des Metallwerkstoffs (1) und/oder einem ähnlichen Fall und/oder der Finite-Elemente-Analyse festgelegt wird.

**8.** Verfahren zur Berechnung einer Restspannung nach einem der Ansprüche 1 bis 4, wobei bei dem Berechnen (S5) in einem Fall, bei dem der berechnete Wert innerhalb einer Fehlerspanne des Messwerts liegt, die Differenz zwischen dem berechneten Wert und dem Messwert als null definiert wird, und in einem Fall, bei dem der berechnete Wert außerhalb der Fehlerspanne des Messwerts liegt, die Differenz zwischen dem berechneten Wert und dem Messwert als eine Differenz zwischen dem berechneten Wert und einer Grenze der Fehlerspanne definiert wird.

**9.** Verfahren zur Berechnung einer Restspannung nach einem der Ansprüche 1 bis 4, wobei als eine Beschränkung zu einem Zeitpunkt, an dem das Optimierungsproblem gelöst wird, eine Bedingung angewendet wird, bei der sich das Volumen des Metallwerkstoffs (1) aufgrund der Erzeugung der Eigenspannung nicht verändert.

**10.** Verfahren zur Berechnung einer Restspannung nach einem der Ansprüche 1 bis 4, wobei es sich bei dem Parameter um die Eigenspannung oder einen Koeffizienten einer Eigenspannungsapproximationsfunktion handelt.

**Revendications**

**1.** Procédé de calcul d'une contrainte résiduelle dans un matériau métallique (1), le procédé utilisant un procédé de déformation inhérente et comprenant :

la préparation (S1) d'une valeur mesurée d'une contrainte résiduelle dans chacune d'une pluralité de portions sur une surface d'un matériau métallique (1) et chacune d'une pluralité de portions sur une face de coupe qui est formée en coupant le matériau métallique (1) ;
la création (S2) d'une pluralité de modèles d'éléments finis ayant chacun une forme identique à celle du matériau métallique (1) après la coupe, le matériau métallique (1) après la coupe incluant une partie de la surface et de la face de coupe ;
le réglage (S3), pour chacun de la pluralité des modèles d'éléments finis créés dans la création (S2), d'un paramètre permettant d'appliquer une déformation inhérente ;
la dérivation (S4), pour chacun de la pluralité des modèles d'éléments finis pour lesquels le paramètre est réglé dans le réglage (S3), d'une valeur calculée de la contrainte résiduelle dans chacune de la pluralité des portions sur la surface et chacune de la pluralité des portions sur la face de coupe, sur la base d'une analyse d'éléments finis ; et
le calcul (S5), pour chacune de la pluralité des portions sur la surface et chacune de la pluralité des portions sur la face de coupe, d'une différence entre la valeur calculée de la contrainte résiduelle dérivée dans la dérivation (S4) et la valeur mesurée de la contrainte résiduelle préparée dans la préparation (S1), dans lequel en tant que problème d'optimisation consistant à minimiser la différence entre la valeur calculée calculée dans le calcul (S5) et la valeur mesurée, après qu'une solution optimale du paramètre est déterminée en répétant le réglage (S3), la dérivation (S4) et le calcul (S5) tout en ajustant le paramètre, une valeur de paramètre à laquelle la solution optimale est déterminée est réglée pour un modèle d'éléments finis ayant une forme identique à celle du matériau métallique (1) avant la coupe, et une contrainte résiduelle est calculée pour le modèle d'éléments finis, sur la base de l'analyse d'éléments finis.

2. Procédé de calcul d'une contrainte résiduelle dans un matériau métallique (1), le procédé utilisant un procédé de déformation inhérente et comprenant :

la préparation (S1) d'une valeur mesurée d'une déformation élastique dans chacune d'une pluralité de portions sur une surface d'un matériau métallique (1) et d'une pluralité de portions sur une face de coupe qui est formée en coupant le matériau métallique (1) ;
la création (S2) d'une pluralité de modèles d'éléments finis ayant chacun une forme identique à celle du matériau métallique (1) après la coupe, le matériau métallique (1) après la coupe incluant une partie de la surface et de la face de coupe ;
le réglage (S3), pour chacun de la pluralité des modèles d'éléments finis créés dans la création (S2), d'un paramètre permettant d'appliquer une déformation inhérente ;
la dérivation (S4), pour chacun de la pluralité des modèles d'éléments finis pour lesquels le paramètre est réglé dans le réglage (S3), d'une valeur calculée de la déformation élastique dans chacune de la pluralité des portions sur la surface et chacune de la pluralité des portions sur la face de coupe, sur la base d'une analyse d'éléments finis ; et
le calcul (S5), pour chacune de la pluralité des portions sur la surface et chacune de la pluralité des portions sur la face de coupe, d'une différence entre la valeur calculée de la déformation élastique dérivée dans la dérivation (S4) et la valeur mesurée de la déformation élastique préparée dans la préparation (S1), dans lequel en tant que problème d'optimisation consistant à minimiser la différence entre la valeur calculée calculée dans le calcul (S5) et la valeur mesurée, après qu'une solution optimale du paramètre est déterminée en répétant le réglage (S3), la dérivation (S4) et le calcul (S5) tout en ajustant le paramètre, une valeur de paramètre à laquelle la solution optimale est déterminée est réglée pour un modèle d'éléments finis ayant une forme identique à celle du matériau métallique (1) avant la coupe, et une contrainte résiduelle est calculée pour le modèle d'éléments finis, sur la base de l'analyse d'éléments finis.

3. Procédé de calcul d'une contrainte résiduelle selon la revendication 1, dans lequel

le matériau métallique (1) comprend une portion d'axe ayant une forme cylindrique et une portion en forme de plaque faisant saillie radialement depuis la circonférence entière de la portion d'axe, et
la surface du matériau métallique (1) comprend une face de congé qui est formée sur une portion de connexion entre la portion d'axe et la portion en forme de plaque.

4. Procédé de calcul d'une contrainte résiduelle selon la revendication 2, dans lequel

le matériau métallique (1) comprend une portion d'axe ayant une forme cylindrique et une portion en forme de plaque faisant saillie radialement depuis la circonférence entière de la portion d'axe, et
la surface du matériau métallique (1) comprend une face de congé qui est formée sur une portion de connexion entre la portion d'axe et la portion en forme de plaque.

5. Procédé de calcul d'une contrainte résiduelle selon la revendication 3 ou 4, dans lequel

la face de congé comprend une région formée en forme d'arc, et
une face étendue de la face de coupe passe par une position centrale d'un arc de la face de congé.

6. Procédé de calcul d'une contrainte résiduelle selon l'une quelconque des revendications 1 à 4, dans lequel, dans le réglage (S3), le paramètre est initialisé sur la base d'au moins un élément parmi une demi-largeur de rayons X diffractés dans la mesure de contrainte par rayons X du matériau métallique (1), la dureté du matériau métallique (1), un cas similaire et l'analyse d'éléments finis.

7. Procédé de calcul d'une contrainte résiduelle selon l'une quelconque des revendications 1 à 4, dans lequel une plage ajustable du paramètre à un moment de la résolution du problème d'optimisation est réglée sous une restriction basée sur au moins un élément parmi une demi-largeur de rayons X diffractés dans la mesure de contrainte par rayons X du matériau métallique (1), la dureté du matériau métallique (1), un cas similaire, et l'analyse d'éléments finis.

**8.** Procédé de calcul d'une contrainte résiduelle selon l'une quelconque des revendications 1 à 4, dans lequel, lors du calcul (S5), dans un cas où la valeur calculée se situe dans une plage d'erreur de la valeur mesurée, la différence entre la valeur calculée et la valeur mesurée est définie comme étant nulle, et dans un cas où la valeur calculée est en dehors de la plage d'erreur de la valeur mesurée, la différence entre la valeur calculée et la valeur mesurée est définie comme étant une différence entre la valeur calculée et une limite de la plage d'erreur.

**9.** Procédé de calcul d'une contrainte résiduelle selon l'une quelconque des revendications 1 à 4, dans lequel on utilise comme restriction à un moment de la résolution du problème d'optimisation une condition dans laquelle le matériau métallique (1) ne change pas de volume en raison de la génération de la déformation inhérente.

**10.** Procédé de calcul d'une contrainte résiduelle selon l'une quelconque des revendications 1 à 4, dans lequel le paramètre est la déformation inhérente ou un coefficient d'une fonction d'approximation de la déformation inhérente.

Start

Preparing step — S1

Creating step — S2

Setting step — S3

Deriving step — S4

Calculating step — S5

Optimal solution

NO

Adjustment of parameter

YES

Calculation of residual stress — S6

End

**FIG. 1**

**FIG. 2**

**FIG. 3**

**FIG. 4**

**FIG. 5**

**FIG. 6**

**EP 3 786 600 B1**

**Patent documents cited in the description**

- JP 2005181172 A

- US 2016273979 A1